# EUROPEAN PATENT APPLICATION

(11) **EP 4 194 543 A1**
(43) Date of publication of application: **14.06.2023**
(21) Application number: 21864756.8
(22) Date of filing: 06.09.2021
(51) Int. Cl.: C12N 1/20, A61K 35/742, A61P 1/02, A23L 33/135, C12R 1/125

(54) **BACILLUS SUBTILIS STRAIN AND USE THEREOF**

(30) Priority: 07.09.2020 KR 20200114170
(71) Applicant: Il Dong Pharmaceutical Co., Ltd., Seoul 06752 (KR)
(72) Inventor: LEE, Byeonghun, Seoul 06752 (KR); KIM, Min Goo, Seoul 06752 (KR); EUN, Suhyeon, Seoul 06752 (KR); KIM, Hyeoungeun, Seoul 06752 (KR); IM, A Rang, Seoul 06752 (KR); HAN, Chiyoung, Seoul 06752 (KR); LEE, Dong Hoon, Seoul 06752 (KR)
(74) Representative: Richardt Patentanwälte PartG mbB
(86) International application number: PCT/KR2021/012053
(87) International publication number: WO 2022/050797

(57) **Abstract**

The present invention relates to a *Bacillus subtilis* ID-A05 strain and a composition including same, and more specifically, to: a novel *Bacillus subtilis* strain effective for both treating cavities and periodontal diseases and alleviating halitosis; a culture medium of the *Bacillus subtilis* strain; a culture supernatant of the *Bacillus subtilis* strain; a pharmaceutical composition for treating cavities and periodontal diseases, the pharmaceutical composition containing, as an active ingredient, the *Bacillus subtilis* strain or a culture medium or culture supernatant thereof; a quasi-drug composition for preventing or alleviating cavities, periodontal diseases, and halitosis, the quasi-drug composition containing, as an active ingredient, the *Bacillus subtilis* strain or a culture medium or culture supernatant thereof, a health functional food composition; and a food composition.

## Description

### TECHNICAL FIELD

This application claims the priority of Korean Patent Application No. 10-2020-01141670, filed on September 7, 2020, the entirety of which is a reference of the present application.

The present invention relates to *Bacillus subtilis* ID-A05 strain and use thereof, and more particularly, to a novel *Bacillus subtilis* strain with all effects of treating cavities and periodontal diseases and improving halitosis, a culture medium of the *Bacillus subtilis* strain, a culture supernatant of the *Bacillus subtilis* strain, a pharmaceutical composition for treating cavities, periodontal diseases or halitosis comprising the *Bacillus subtilis* strain, or a culture medium or culture supernatant thereof as an active ingredient, and a quasi-drug composition, a health functional food composition and a food composition for preventing or improving cavities, periodontal diseases or halitosis comprising the *Bacillus subtilis* strain, or a culture medium or culture supernatant thereof as an active ingredient.

### BACKGROUND ART

Recently, due to incorrect eating habits and aging, immunity is lowered and nutrition and metabolic disorders increase, and as a result, the number of patients with periodontal diseases has been increased. Direct causes of periodontal disease are a bacterial membrane called plaque, which is constantly formed in the teeth, and scale which is hardened without removing the plaque. When the plaque and the scale are accumulated, the gums are detached from the teeth to cause inflammation between the teeth and the gums.

Periodontal disease refers to symptoms associated with inflammatory diseases that destroy soft tissues such as periodontal ligaments and gingiva surrounding teeth and hard tissues such as alveolar bone.

When the alveolar bone and the like are congenitally and acquiredly degraded as the age increases, periodontal diseases will be developed, and the representative periodontal diseases include gingivitis and periodontal inflammation, alveolar bone osteodystrophy, and the like. The gingivitis is an initial periodontal disease in which inflammation occurs in the soft tissue of the gums, and the symptom is relatively light and recovery is in a reversible state. The periodontal inflammation is a case where the gingivitis is not treated and the inflammation is progressed around the gums and the pubic bone, and when chronic inflammation response stimulated by the toxin of bacteria progresses, the gap between the gums and teeth is separated and an infected periodontal pocket is formed. As the periodontal inflammation is severe, the depth of the periodontal pocket is deepened, and eventually, inflammation is caused in the periodontal ligament, and the bone loss occurs. In addition, when bone resorption exceeds bone formation due to various factors in the metabolism of bone formation by osteoblasts and bone resorption by osteoclasts in alveolar bone, bone mass decreases below the limit, and alveolar bone formation disorders such as alveolar osteoporosis occur.

In the periodontal diseases, the prevention is most important through the improved oral hygiene establishment of the patients, and in the clinical, the surgical treatment such as non-surgical or surgical scaling, root planning, gingival curettage, and regeneration of periodontal tissue applied with new attachment is mainly conducted. However, in these surgical treating methods, there are most cases that treatment is troublesome, effective treatment is difficult, the treatment is limited to the treatment when the disease is proceeding to some extent instead of the prevention of the disease, and the disease is chronically progressed when the treatment is not performed. Further, as additional treatment, the taking of systemic antibiotics and local sustained release agents have been used, but even in unnecessary sites, the drug is excessively delivered, resulting in side effects, and recently, it has been reported that periodontal disease bacteria, which exhibit antibiotic resistance, cause serious problems.

The cavity refers to a decayed tooth and a symptom in which the hard tissue of the teeth is eroded and lost, and is referred to as dental caries. The cavity is an infectious bacterial disease which causes the pain in which a part of the hard calcified tissue of the tooth is dissolved and destroyed, resulting in losing the tooth, and occupies the largest part of dental diseases and the incidence is increasing. According to the report of the Korean Dental Association, 90% or more of children experienced the dental caries, and 80% or more of adults have gum diseases. The main cause of these diseases is infection by microorganisms in the mouth induced by the interaction of bacteria, food and saliva.

Meanwhile, halitosis refers to the smell derived from the mouth and vicinal organs, and many people appeal the inconvenience by halitosis in everyday life. 85% to 90% of halitosis is derived from the mouth and particularly, derived from the back of the tongue. The main ingredient of halitosis is volatile sulfur compounds, and90% of the total amount of volatile sulfur compounds consists of hydrogen sulfide made of cysteine and methyl mercaptan made of methionine. These ingredients are generated by anaerobic bacteria, and the back of the tongue becomes the most important habitat. The site is a place which is not very well cleaned by the saliva, and has many small depressions where the bacteria are constantly living. Production of the volatile sulfur compounds by anaerobic bacteria is most important as the cause of halitosis, but also caused by other oral diseases such as cavities and periodontal inflammation.

Bacteria frequently found in the mouth of patients with halitosis are *Fusobacterium nucleatum, Prevotella intermedia,* and *Porphyromonas gingivalis.* In the related art, Bacteroides and Porphyromonas are similar bacteria that were known as the same genus along with Prevotella. Many kinds of anaerobic bacteria causing halitosis are involved, but representative bacteria causing halitosis are *Fusobacterium nucleatum* and *Porphyromonas gingivalis.*

In recent years, there was an attempt to inhibit the proliferation of anaerobic bacteria *in vitro* using lactic acid bacteria. The lactic acid bacteria are bacteria which produce lactic acid as a final metabolic product by fermenting carbohydrates and the lactic acid bacteria are present in the mouth and digestive tract of humans and animals, and are typically used in the manufacturing process of fermented foods such as kimchi or yogurt. However, while the lactic acid bacteria are diluted by the saliva, like metal salts or disinfectants when administered to the mouth, the lactic acid bacteria are swallowed right away, so that there is a problem that it is difficult for the lactic acid bacteria to remain in the mouth. In addition, lactic acid bacteria such as *Lactobacillus acidophilus* and *Lactobacillus casei* make strong acid to inhibit the proliferation of anaerobic bacteria *in vitro,* but in the mouth of the healthy human, the strong acid made by the lactic acid bacteria is neutralized by a buffer action of the saliva, and thus, it is difficult to inhibit the proliferation of other bacteria. Moreover, since the strong acid causes the dental caries, when these bacteria are administered in the mouth for a long time, there is a problem that it is not good for oral hygiene.

On the other hand, there was a strain having one function of improvement of cavities, improvement of periodontal diseases, and improvement of halitosis in the related art, but there is almost no strain having all functions of improving cavities, periodontal diseases, and halitosis.

### PRIOR ARTS

### PATENT DOCUMENT

(Patent Document 1) Korean Patent Registration No. 10-1956759
(Patent Document 2) Korean Patent Registration No. 10-1629551

### DISCLOSURE

### TECHNICAL PROBLEM

Therefore, the present inventors found that a specific *Bacillus subtilis* strain had an excellent effect of treating or preventing cavities and periodontal diseases and an excellent effect of improving halitosis while making efforts to find strains exhibiting an excellent activity to treatment of periodontal diseases and then completed the present invention.

Accordingly, an object of the present invention is to provide a *Bacillus subtilis* ID-A05 strain (Accession No.: KCTC14184BP).

Another object of the present invention is to provide a culture medium of the *Bacillus subtilis* ID-A05 strain.

Yet another object of the present invention is to provide a culture supernatant of the *Bacillus subtilis*ID-A05 strain.

Another object of the present invention is to provide a pharmaceutical composition for treating cavities, periodontal diseases or halitosis comprising a *Bacillus subtilis* ID-A05 strain, a culture medium thereof, or a culture supernatant thereof as an active ingredient.

In addition, another object of the present invention is to provide a pharmaceutical composition for treating cavities, periodontal diseases or halitosis consisting of a *Bacillus subtilis* ID-A05 strain, a culture medium thereof, or a culture supernatant thereof.

In addition, another object of the present invention is to provide a pharmaceutical composition for treating cavities, periodontal diseases or halitosis essentially consisting of a *Bacillus subtilis* ID-A05 strain, a culture medium thereof, or a culture supernatant thereof.

Another object of the present invention is to provide a quasi-drug composition for preventing or improving cavities, periodontal diseases or halitosis comprising a *Bacillus subtilisID-A05* strain, a culture medium thereof, or a culture supernatant thereof as an active ingredient.

In addition, another object of the present invention is to provide a quasi-drug composition for preventing or improving cavities, periodontal diseases or halitosis consisting of a *Bacillus subtilis* ID-A05 strain, a culture medium thereof, or a culture supernatant thereof.

In addition, another object of the present invention is to provide a quasi-drug composition for preventing or improving cavities, periodontal diseases or halitosis essentially consisting of a *Bacillus subtilis* ID-A05 strain, a culture medium thereof, or a culture supernatant thereof.

Another object of the present invention is to provide a health functional food composition for preventing or improving cavities, periodontal diseases or halitosis comprising a *Bacillus subtilis* ID-A05 strain, a culture medium thereof, or a culture supernatant thereof as an active ingredient.

In addition, another object of the present invention is to provide a health functional food composition for preventing or improving cavities, periodontal diseases or halitosis consisting of a *Bacillus subtilis*ID-A05 strain, a culture medium thereof, or a culture supernatant thereof.

In addition, another object of the present invention is to provide a health functional food composition for preventing or improving cavities, periodontal diseases or halitosis essentially consisting of a *Bacillus subtilis* ID-A05 strain, a culture medium thereof, or a culture supernatant thereof.

Another object of the present invention is to provide a food composition for preventing or improving cavities, periodontal diseases or halitosis comprising a *Bacillus subtilis*ID-A05 strain, a culture medium thereof, or a culture supernatant thereof as an active ingredient.

In addition, another object of the present invention is to provide a food composition for preventing or improving cavities, periodontal diseases or halitosis consisting of a *Bacillus subtilis* ID-A05 strain, a culture medium thereof, or a culture supernatant thereof.

In addition, another object of the present invention is to provide a food composition for preventing or improving cavities, periodontal diseases or halitosis essentially consisting of a *Bacillus subtilis* ID-A05 strain, a culture medium thereof, or a culture supernatant thereof.

Another object of the present invention is to provide a toothpaste composition for preventing or improving cavities, periodontal diseases or halitosis comprising a *Bacillus subtilis* strain, a culture medium thereof, or a culture supernatant thereof as an active ingredient.

In addition, another object of the present invention is to provide a toothpaste composition for preventing or improving cavities, periodontal diseases or halitosis consisting of a *Bacillus subtilis* strain, a culture medium thereof, or a culture supernatant thereof.

In addition, another object of the present invention is to provide a toothpaste composition for preventing or improving cavities, periodontal diseases or halitosis essentially consisting of a *Bacillus subtilis* strain, a culture medium thereof, or a culture supernatant thereof.

Another object of the present invention is to provide a mouth washer composition for preventing or improving cavities, periodontal diseases or halitosis comprising a *Bacillus subtilis* strain, a culture medium thereof, or a culture supernatant thereof as an active ingredient.

In addition, another object of the present invention is to provide a mouth washer composition for preventing or improving cavities, periodontal diseases or halitosis consisting of a *Bacillus subtilis* strain, a culture medium thereof, or a culture supernatant thereof.

In addition, another object of the present invention is to provide a mouth washer composition for preventing or improving cavities, periodontal diseases or halitosis essentially consisting of a *Bacillus subtilis* strain, a culture medium thereof, or a culture supernatant thereof.

Another object of the present invention is to provide a gargle composition for preventing or improving cavities, periodontal diseases or halitosis comprising a *Bacillus subtilis* strain, a culture medium thereof, or a culture supernatant thereof as an active ingredient.

In addition, another object of the present invention is to provide a gargle composition for preventing or improving cavities, periodontal diseases or halitosis consisting of a *Bacillus subtilis* strain, a culture medium thereof, or a culture supernatant thereof.

In addition, another object of the present invention is to provide a gargle composition for preventing or improving cavities, periodontal diseases or halitosis essentially consisting of a *Bacillus subtilis* strain, a culture medium thereof, or a culture supernatant thereof.

Another object of the present invention is to provide use of a *Bacillus subtilis* strain, a culture medium thereof, or a culture supernatant thereof for preparing an agent for treating cavities, periodontal diseases or halitosis.

Another object of the present invention is to provide a method of treating cavities, periodontal diseases or halitosis comprising administering an effective amount of a composition comprising a *Bacillus subtilis* strain, a culture medium thereof, or a culture supernatant thereof to a subject in need thereof.

### TECHNICAL SOLUTION

To achieve the object of the present invention, the present invention provides a *Bacillus subtilis* ID-A05 (Accession No.: KCTC14184BP) strain.

To achieve another the object of the present invention, the present invention provides a culture medium of the *Bacillus subtilis*ID-A05 strain.

To achieve another the object of the present invention, the present invention provides a culture supernatant of the *Bacillus subtilis* ID-A05 strain.

To achieve another the object of the present invention, the present invention provides a pharmaceutical composition for treating cavities, periodontal diseases or halitosis comprising a *Bacillus subtilis*ID-A05 strain, a culture medium thereof, or a culture supernatant thereof as an active ingredient.

In addition, the present invention provides a pharmaceutical composition for treating cavities, periodontal diseases or halitosis consisting of a *Bacillus subtilis* ID-A05 strain, a culture medium thereof, or a culture supernatant thereof

In addition, the present invention provides a pharmaceutical composition for treating cavities, periodontal diseases or halitosis essentially consisting of a *Bacillus subtilis* ID-A05 strain, a culture medium thereof, or a culture supernatant thereof.

To achieve another the object of the present invention, the present invention provides a quasi-drug composition for preventing or improving cavities, periodontal diseases or halitosis comprising a *Bacillus subtilis* ID-A05 strain, a culture medium thereof, or a culture supernatant thereof as an active ingredient.

In addition, the present invention provides a quasi-drug composition for preventing or improving cavities, periodontal diseases or halitosis consisting of a *Bacillus subtilis* ID-A05 strain, a culture medium thereof, or a culture supernatant thereof.

In addition, the present invention provides a quasi-drug composition for preventing or improving cavities, periodontal diseases or halitosis essentially consisting of a *Bacillus subtilis* ID-A05 strain, a culture medium thereof, or a culture supernatant thereof.

To achieve another the object of the present invention, the present invention provides a health functional food composition for preventing or improving cavities, periodontal diseases or halitosis comprising a *Bacillus subtilis* ID-A05 strain, a culture medium thereof, or a culture supernatant thereof as an active ingredient.

In addition, the present invention provides a health functional food composition for preventing or improving cavities, periodontal diseases or halitosis consisting of a *Bacillus subtilis* ID-A05 strain, a culture medium thereof, or a culture supernatant thereof.

In addition, the present invention provides a health functional food composition for preventing or improving cavities, periodontal diseases or halitosis essentially consisting of a *Bacillus subtilis* ID-A05 strain, a culture medium thereof, or a culture supernatant thereof.

To achieve another the object of the present invention, the present invention provides a food composition for preventing or improving cavities, periodontal diseases or halitosis comprising a *Bacillus subtilis* ID-A05 strain, a culture medium thereof, or a culture supernatant thereof as an active ingredient.

In addition, the present invention provides a food composition for preventing or improving cavities, periodontal diseases or halitosis consisting of a *Bacillus subtilis* ID-A05 strain, a culture medium thereof, or a culture supernatant thereof.

In addition, the present invention provides a food composition for preventing or improving cavities, periodontal diseases or halitosis essentially consisting of a *Bacillus subtilis* ID-A05 strain, a culture medium thereof, or a culture supernatant thereof.

To achieve another the object of the present invention, the present invention provides a toothpaste composition for preventing or improving cavities, periodontal diseases or halitosis comprising a *Bacillus subtilis* strain, a culture medium thereof, or a culture supernatant thereof as an active ingredient.

In addition, the present invention provides a toothpaste composition for preventing or improving cavities, periodontal diseases or halitosis consisting of a *Bacillus subtilis* strain, a culture medium thereof, or a culture supernatant thereof.

In addition, the present invention provides a toothpaste composition for preventing or improving cavities, periodontal diseases or halitosis essentially consisting of a *Bacillus subtilis* strain, a culture medium thereof, or a culture supernatant thereof.

To achieve another the object of the present invention, the present invention provides a mouth washer composition for preventing or improving cavities, periodontal diseases or halitosis comprising a *Bacillus sublilis* strain, a culture medium thereof, or a culture supernatant thereof as an active ingredient.

In addition, the present invention provides a mouth washer composition for preventing or improving cavities, periodontal diseases or halitosis consisting of a *Bacillus subtilis* strain, a culture medium thereof, or a culture supernatant thereof.

In addition, the present invention provides a mouth washer composition for preventing or improving cavities, periodontal diseases or halitosis essentially consisting of a *Bacillus subtilis* strain, a culture medium thereof, or a culture supernatant thereof.

To achieve another the object of the present invention, the present invention provides a gargle composition for preventing or improving cavities, periodontal diseases or halitosis comprising a *Bacillus subtilis* strain, a culture medium thereof, or a culture supernatant thereof as an active ingredient.

In addition, the present invention provides a gargle composition for preventing or improving cavities, periodontal diseases or halitosis consisting of a *Bacillus subtilis* strain, a culture medium thereof, or a culture supernatant thereof.

In addition, the present invention provides a gargle composition for preventing or improving cavities, periodontal diseases or halitosis essentially consisting of a *Bacillus subtilis* strain, a culture medium thereof, or a culture supernatant thereof.

To achieve another the object of the present invention, the present invention provides use of a *Bacillus subtilis* strain, a culture medium thereof, or a culture supernatant thereof for preparing an agent for treating cavities, periodontal diseases or halitosis.

To achieve another the object of the present invention, the present invention provides a method of treating cavities, periodontal diseases or halitosis comprising administering an effective amount of a composition containing a *Bacillus subtilis* strain, a culture medium thereof, or a culture supernatant thereof to a subject in need thereof.

Unless otherwise defined, all technical and scientific terms used herein have the same meanings that are commonly understood by those skilled in the art. The following references provide one of skills with the general definition of various terms used in the specification of the present invention: Singleton et al., DICTIONARY OF MICROBIOLOGY AND MOLECULAR BIOLOGY(2th ed. 1994); THE CAMBRIDGE DICTIONARY OF SCIENCE AND TECHNOLOGY(Walkered., 1988); and Hale & Marham, THE HARPER COLLINS DICTIONARY OF BIOLOGY.

Hereinafter, the present invention will be described in detail.

The present invention provides a *Bacillus subtilis* ID-A05 strain (Accession No.: KCTC14184BP).

The strain may be at least one selected from the group consisting of a strain itself, a strain lysate, a strain concentrate, a strain extract and a strain dried product.

In the present invention, the term 'Bacillus' refers to a genus of aerobic or facultative anaerobic gram-positive bacilli which is widely distributed in the nature, and microorganisms belonging to the genus bacillus include *Bacillus velezensis, Bacillus subtilis,* and the like. The present inventors identified a novel strain belonging to *Bacillus subtilis* as bacillus bacteria with an effect of treating or preventing cavities and periodontal diseases as follows.

*Bacillus subtilis* ID-A05 (accession number KCTC14184BP) strain according to the present invention is a novel *Bacillus subtilis* strain derived from fermented food. Although the *Bacillus subtilis* ID-A05 strain was isolated and identified from fermented food in the present invention, the acquisition route is not limited thereto.

As a result of 16S rDNA sequencing analysis for identification and classification of microorganisms, it was confirmed that the strain isolated from fermented food through the following examples was 99% identical to *Bacillus subtilis*. *Bacillus subtilis* ID-A05 strain was deposited with the Korea Research Institute of Bioscience and Biotechnology on May 11, 2020 (accession number: KCTC14184BP).

The culture supernatant of the *Bacillus subtilis* ID-A05 strain of the present invention is characterized by having an antibacterial effect against the causative bacteria of periodontal disease, an inhibitory effect on inflammatory cytokine secretion, and an inhibitory effect on biofilm production as well as an inhibitory effect on volatile sulfur compounds (VSC), which are the main cause of halitosis.

This is well shown in embodiments of the present invention.

In one embodiment of the present invention, the culture supernatant of the novel *Bacillus subtilis* ID-A05 strain isolated from fermented food was inoculated into *Porphyromonas gingivalis* (PGI), a representative causative bacterium of periodontal disease, and TYB medium, and then the absorbance was measured. As a result, it was confirmed that the culture supernatant of the *Bacillus subtilis* ID-A05 strain had an excellent antibacterial effect against PGI (see Example 5).

Further, in another embodiment of the present invention, in order to confirm the biofilm inhibitory effect of the culture supernatants of the *Bacillus subtilis* ID-A05 strain, a medium was dispensed in a 96 well plate coated with saliva (4 hr, 37°C) and then *Porphyromonas gingivalis* (PGI) and *Fusobacterium nucleatum* (FNU) were inoculated at concentrations of 25% and 0.5%, respectively, and samples were simultaneously treated and anaerobic-cultured at 37°C for 4 days, and the result thereof was confirmed. As a result, it was confirmed that the culture supernatant of the *Bacillus subtilis* ID-A05 strain had an excellent biofilm inhibitory effect (see Examples 6 and 7).

Further, in another embodiment of the present invention, in order to confirm the inflammatory cytokine secretion inhibitory effect of the culture supernatants of the *Bacillus subtilis* ID-A05 strain, HGF-1 cells were seeded with 1×10⁴ cells/well on a 96 well plate and cultured for 24 hours, a candidate group was treated for 24 hours and then treated with 50 µg/ml *of P. gingivalis*-LPS for 24 hours, and the supernatants were collected and analyzed. As a result, it was confirmed that the *Bacillus subtilis* ID-A05 strain suppresses the secretion of inflammatory cytokines (see Example 9).

The present invention provides a culture medium of *Bacillus subtilis* ID-A05 strain.

The term "culture medium" of the present invention refers to a liquid medium in which the *Bacillus subtilis* ID-A05 strain of the present invention are cultured and comprises a tryptic soy broth (TSB) with strains.

The present invention provides a culture supernatant of a novel *Bacillus subtilis* strain.

The term "culture supernatant" of the present invention refers to a liquid medium in which the *Bacillus subtilis* ID-A05 strain of the present invention are cultured and then the cells are removed, and comprises a tryptic soy broth (TSB) without cells.

The 'liquid medium' is a liquid medium without agar, and is used for proliferation of microorganisms and for biochemical testing, proliferation, and detecting metabolites of bacteria.

The isolated *bacillus subtilis* strains were cultured using a shaking culture method. The shaking culture method is a method of effectively ventilating and stirring through continuously shaking by fixing a triangular flask or the like to a reciprocal shaker or a rotary shaker. In the case of the shaking culture, the strains were cultured for 40 hours under a condition of 37°C and 200 rpm in a TSB medium (MB cell) containing tryptone 17 g/L, soytone 3 g/L, dextrose 2.5 g/L, sodium chloride 5 g/L, and dipotassium phosphate 2.5 g/L. After completion of the culture, the culture medium was centrifuged at 13,000 rpm for 20 minutes to remove the cells and obtain a culture supernatant. The pH of the culture supernatant was corrected to 6.5 using 8.25% HCl, and then the culture supernatant was antibacterialized using a 0.22 µm filter for complete cell removal.

In order to achieve another object of the present invention, the present invention provides a pharmaceutical composition for treating cavities, periodontal diseases or halitosis comprising a *Bacillus subtilis* ID-A05 strain, a culture medium thereof, or a culture supernatant thereof as an active ingredient.

The term "treatment" of the present invention means approach for obtaining a beneficial or preferable clinical result. For the object of the present invention, the beneficial or preferable clinical result includes unrestrictively alleviation of symptoms, reduction of a disease range, stabilization (that is, not degraded) of disease conditions, delay or decreased speed of disease progression, improvement or temporary alleviation and mitigation of disease conditions (partially or overall), and whether to be detectable or not. In addition, the 'treatment' refers to both therapeutic therapy and preventive or precaution methods. The above treatments include not only preventive disorders, but also the treatment required for the disorders already occurring.

The term "cavities" of the present invention mean that the enamel of teeth protecting the tooth dentin is damaged by acid generated while sugar, starch, and the like are decomposed by bacteria that habitat in the mouth.

The term 'periodontal diseases' of the present invention may be at least one selected from the group consisting of gingivitis, periodontal inflammation, gingival bleeding, gingival retraction, periodontal pocket, pericoronitis and periodontal abscess, preferably gingivitis, but is not limited thereto.

The term 'biofilm' of the present invention refers to a phenomenon in which various microorganisms, including bacteria in plaque, are coagulated with each other, and when the biofilm is not wiped to become thick and the amount of pathological bacteria therein increases to be unaffordable for the defense of our body, oral diseases occur.

The pharmaceutical composition according to the present invention may be formulated in a suitable form containing a *Bacillus subtilis* strain or a culture medium or culture supernatant thereof alone or in combination with a pharmaceutically acceptable carrier and may further contain an excipient or a dilute. The "pharmaceutically acceptable" refers to a nontoxic composition that is physiologically acceptable and does not cause allergic reactions, such as gastrointestinal disorder, dizziness, etc., or similar reactions thereto when administered to humans.

The pharmaceutically acceptable carrier may further include, for example, a carrier for oral administration or a carrier for parenteral administration. The carrier for oral administration may include lactose, starch, cellulose derivatives, magnesium stearate, stearic acid, and the like. In addition, the carrier for oral administration may include various drug delivery materials used for oral administration to peptide preparations. In addition, the carrier for parenteral administration may include water, suitable oil, saline, aqueous glucose, glycol, and the like, and may further include a stabilizer and a preservative. A suitable stabilizer includes an antioxidant such as sodium hydrogen sulfite, sodium sulfite or ascorbic acid. A suitable preservative includes benzalkonium chloride, methyl- or propyl-paraben and chlorobutanol. The pharmaceutical composition of the present invention may further include a lubricant, a wetting agent, a sweetening agent, a flavoring agent, an emulsifying agent, a suspending agent, and the like in addition to the above ingredients. Other pharmaceutically acceptable carriers and agents may refer to those known in the art.

The composition of the present invention may be administered to mammals including humans even by any method. For example, the composition of the present invention may be orally or parenterally administered. The parenteral administration method is not limited thereto, but may be intravenous, intramuscular, intraarterial, intramedullary, intrathecal, intracardiac, transdermal, subcutaneous, intraperitoneal, intranasal, intestinal tract, topical, sublingual, or intrectal administration.

The pharmaceutical composition of the present invention may be formulated as a preparation for oral administration or parenteral administration according to the route of administration as described above.

In the case of the preparation for oral administration, the composition of the present invention may be formulated using methods known in the art with an orally disintegrating film agent, powder, granules, tablets, pills, sugar-coating tablets, capsules, liquids, gels, syrups, a slurry agent, suspensions, and the like. For example, the oral preparation may be obtained as tablets or sugar-coating tablets by mixing an active ingredient with a solid excipient, pulverizing the mixture, adding a suitable adjuvant, and then processing the mixture into a granule mixture. An example of the suitable excipient may include fillers such as sugars including lactose, dextrose, sucrose, sorbitol, mannitol, xylitol, erythritol and maltitol, etc., starches including corn starch, wheat starch, rice starch, potato starch, etc., celluloses including cellulose, methylcellulose, sodium carboxymethylcellulose, hydroxypropylmethyl-cellulose, etc., gelatin, polyvinylpyrrolidone, and the like. In some cases, crosslinked polyvinylpyrrolidone, agar, alginic acid or sodium alginate may be added as a disintegrant. In addition, the pharmaceutical composition of the present invention may further include an anti-coagulating agent, a lubricant, a wetting agent, a flavoring, emulsifiers, an antiseptic, and the like.

The preparation for parenteral administration may be formulated in a form of a patch agent, an injection, a cream agent, a lotion agent, an external ointment agent, an oil agent, a moisturizer, a gel agent, an aerosol, and a nasal suction agent by methods known in the art. These formulations are described in all prescriptions which are generally known in the pharmaceutical chemistry.

In order to achieve another object of the present invention, the present invention provides a quasi-drug composition for preventing or improving cavities, periodontal diseases or halitosis comprising a *Bacillus subtilis* ID-A05 strain, a culture medium thereof, or a culture supernatant thereof as an active ingredient.

In an embodiment of the present invention, in order to confirm an effect on halitosis of the culture supernatants of the *Bacillus subtilis* ID-A05 strain isolated from fermented foods, generation inhibitory activity of volatile sulfur compounds (VSC) as a main cause of halitosis was tested. As a result, it was confirmed that the culture supernatants of the *Bacillus subtilis* ID-A05 strain had an excellent VSC generation inhibitory effect (see Example 8).

The term 'halitosis' of the present invention is a smell that is derived from mouth and vicinal organs, which usually give displeasure to others or yourself. The main cause of halitosis is volatile sulfur compounds (VSC) that are produced by degrading amino acids and proteins contained in food residue, saliva, blood, oral mucous membrane cells, and the like by bacteria present in the mouth. When the volatile compounds are analyzed, methyl mercaptan (CH₃SH) and hydrogen sulfide (H₂S) account for 90%, which smell of rotten egg and rotten onion.

The 'improvement' of the present invention means all actions that at least reduce parameters associated with alleviation or treatment of conditions, for example, the degree of symptoms.

The term "quasi-drug" of the present invention comprisesproducts corresponding to one of fibers, rubber products, or those similar thereto used for treating, alleviating, healing or preventing diseases of humans or animals, those which are weakly act on the human body or do not directly act on the human body and are not mechanisms or machines and similar thereto, and preparations used for sterilization, deinsectization and similar purposes thereto for prevention of infectious diseases.

In the present invention, the quasi-drug may be at least one selected from the group consisting of a mouth washer, a gargle, an anti-liquid agent, a toothpaste, a sanitary pad, a disposable liner, a mask, an eye bandage, a bandage, a resilient bandage, a gypsum bandage, a cylindrical elastic bandage, a gauze, cotton wool, a bandaid, an anti-odor agent, an ointment, and cataplasmas, but is not limited thereto.

In order to achieve another object of the present invention, the present invention provides a health functional food composition for preventing or improving cavities, periodontal diseases or halitosis comprising a *Bacillus subtilis* ID-A05 strain, a culture medium thereof, or a culture supernatant thereof as an active ingredient.

The term "health functional food" of the present invention comprises food groups, which give added values to the food so as to act and express the functions of the corresponding food for a specific purpose using physical, biochemical, biotechnological methods, or foods designed and processed to sufficiently express internal regulation functions for bio-defensive rhythm regulation, prevention and recovery of diseases, etc. of the food composition.

The health functional food composition of the present invention may be used for treating or preventing cavities and periodontal diseases or improving halitosis.

In the present invention, the health functional food composition may be at least one formulation selected from formulations consisting of tablets, capsules, pills, and granules, but is not limited thereto.

In addition, the composition may be formulated and used in the form of oral formulations such as powders, granules, tablets, capsules, suspensions, emulsions, syrups, aerosols, etc., external preparations, suppositories, and sterile injectable solutions according to each general method. The carrier, the excipient, and the diluent, which may be included in the composition including compounds, may include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil. In the case of the formulations, the formulations may be prepared by using a diluent or an excipient, such as a filler, an extender, a binder, a wetting agent, a disintegrating agent, and a surfactant which are generally used. A solid formulation for oral administration includes a tablet, a pill, a powder, a granule, a capsule, and the like, and the solid formulation may be prepared by mixing at least one excipient, for example, starch, calcium carbonate, sucrose or lactose, gelatin, and the like with the extract. Further, lubricants such as magnesium stearate, talc may be used in addition to simple excipients. A liquid formulation for oral administration corresponds to a suspension, an oral liquid, an emulsion, a syrup, and the like, and may include various excipients, for example, a wetting agent, a sweetening agent, an aromatic agent, a preserving agent, and the like in addition to water and liquid paraffin which are commonly used simple diluents. Formulations for parenteral administration include a sterile aqueous solution, a non-aqueous solution, a suspension, an emulsion, a lyophilizing agent, and a suppository. As the non-aqueous solution and the suspension, propylene glycol, polyethylene glycol, vegetable oil such as olive oil, injectable ester such as ethyl oleate, and the like may be used. As a base of the suppository, witepsol, macrogol, tween 61, cacao butter, laurinum, glycerogelatin, and the like may be used.

In order to achieve another object of the present invention, the present invention provides a food composition for preventing or improving cavities, periodontal diseases or halitosis comprising a *Bacillus subtilis* ID-A05 strain, a culture medium thereof, or a culture supernatant thereof as an active ingredient.

The food composition according to the present invention may be prepared in a composition form in combination with known active ingredients which are known to be effective in cavities, periodontal diseases or halitosis and includes all forms such as nutritional supplements, health food, food additives, and the like. The types of food composition may be prepared in various forms according to a general method known in the art. For example, the health foods may be prepared in forms of teas, juices, and drinks containing the food composition of the present invention and may be taken by granulating, encapsulating or powdering the food composition comprising the Bacillus velezensis strain or the culture medium or culture supernatant thereof as the active ingredient.

Further, the health functional foods may be prepared by adding the food composition of the present invention to beverages (including alcoholic beverages), fruits and processed foods thereof (e.g., canned fruit, bottled food, jam, marmalade, etc.), fish, meat and processed foods thereof (e.g., ham, sausage, corned beef, etc.), bread and noodles (e.g., udon, buckwheat noodles, ramen, spaghetti, macaroni, etc.), fruit juice, various drinks, cookies, sweets, dairy products (e.g., butter, cheese, etc.), edible vegetable oil, margarine, vegetable protein, retort food, frozen food, various seasonings (e.g., soybean paste, soy sauce, sauce, etc.), etc.

In the food composition of the present invention, in addition to the Bacillus velezensis strain or the culture medium or culture supernatant thereof, as auxiliary ingredients, mineral-containing compounds such as vitamins B, C, and E, beta-carotene, Ca, Mg, and Zn, and compounds such as phospholipids such as lecithin or maltol, oligosaccharides, amino acids, and the like may be used. Among them, it is more preferable because it is possible to reinforce a bioactivity effect by mixing and using 2 to 3 ingredients of vitamins C and E, beta-carotene, maltol, and the like.

Further, in addition to the ingredients, for tempting, known additives may be used by mixing natural flavors or natural juices such as plum flavor, lemon flavor, pineapple flavor or herb flavor, natural pigments such as chlorophyllin and flavonoid, sweetening ingredients fructose, honey, sugar alcohol, and sugar, and acidifiers such as citric acid and sodium citrate.

In the food composition of the present invention, a preferable content of the *Bacillus subtilis* strain or the culture medium or culture supernatant thereof may be about 0.01 to 10 wt% with respect to the total weight of the food. Preferably, when the food composition is taken in an amount of 0.1 mg/kg/day to 200 mg/kg/day, an effect of preventing or improving cavities, periodontal diseases or halitosis may be exhibited.

The present invention provides use of a *Bacillus subtilis* strain, a culture medium thereof, or a culture supernatant thereof for preparing an agent for treating cavities, periodontal diseases or halitosis.

The present invention provides a method of treating cavities, periodontal diseases or halitosis comprising administering an effective amount of a composition containing a *Bacillus subtilis* strain, a culture medium thereof, or a culture supernatant thereof to a subject in need thereof.

The term 'effective amount' of the present invention means an amount which exhibits effects of improving, treating, preventing, detecting, and diagnosing cavities, periodontal diseases or halitosis, or inhibiting or reducing cavities, periodontal diseases or halitosis when administered to the subject. The 'subject' may be animals, preferably, mammals, particularly animals including humans and may also be cells, tissues, and organs derived from animals. The subject may be a patient requiring the effects.

The term 'treatment' of the present invention comprehensively refers to improving cavities, periodontal diseases or halitosis or symptoms of these diseases, and may include treating or substantially preventing these diseases or improving the conditions thereof and includes alleviating, treating or preventing a symptom or most of symptoms derived from the diseases, but is not limited thereto.

The term "comprising' herein is used in the same meaning as 'including' or 'characterized by', and does not exclude additional ingredients or steps of the method which are not specifically mentioned in the composition or the method according to the present invention. The term 'consisting of' means excluding additional elements, steps or ingredients, etc., unless otherwise described. The term 'essentially consisting of' means including substances or steps which do not substantially affect basic properties thereof in addition to the described substances or steps within the range of the composition or the method.

### ADVANTAGEOUS EFFECTS

According to the present invention, the composition comprising the *Bacillus subtilis* ID-A05 strain, the culture media or culture supernatants thereof exhibit antibacterial, anti-inflammatory, and biofilm inhibitory effects on causative bacteria of cavities and periodontal diseases to have an excellent effect of preventing or treating cavities and periodontal diseases or improving halitosis.

### DESCRIPTION OF DRAWINGS

FIG. 1 illustrates an antibacterial test result for *Porphyromonas gingivalis* (PGI).
FIG. 2 illustrates a result of evaluating biofilm efficacy for samples of strains which had an excellent effect in antibacterial evaluation of *Porphyromonas gingivalis* (PGI).
FIG. 3 illustrates a result of measuring a biofilm inhibitory effect using a scanning electronic microscope (SEM).
FIG. 4 illustrates a result of evaluating biofilm removal efficacy.
FIGS. 5a to 5c illustrate inhibitory efficacy results of three cytokines IL-1β, IL-6, and TNF-α by LPS secreted from *Porphyromonas gingivalis* (PGI).
FIG. 6 illustrates a result of testing VSC generation inhibitory activity.
FIG. 7 illustrates an antibacterial test result for *Streptococcus mutans* (SMU).
FIG. 8 illustrates a result of evaluating *Streptococcus mutans* (SMU)-derived biofilm formation inhibitory efficacy.
FIG. 9 illustrates a result of evaluating *Streptococcus mutans* (SMU)-derived biofilm formation inhibitory efficacy using a scanning electronic microscopy (SEM).

### MODES FOR THE INVENTION

Hereinafter, the present invention will be described in detail.

However, the following Examples are just illustrative of the present invention, and the contents of the present invention are not limited to the following Examples.

### Example 1: Isolation of Bacillus subtilis

A liquid of fermented foods was diluted with a multistage dilution method using a sterile saline solution, smeared on a solid medium, and cultured at 37°C for 2 to 3 days. The respective colonies on the solid medium were cultured in a liquid medium at 37°C for 2 to 3 days. The purely isolated strains were identified by sequencing and whole-genome sequencing of 16S rDNA through Macrogen Co., Ltd. (Seoul, Korea).

### Example 2: 16s rDNA identification of Bacillus subtilis

Genomic DNA extraction was performed using an QIAamp Fast DNA Stool Mini Kit (Qiagen, Germany). Base sequence amplification of 16S rDNA was performed by a polymerase chain reaction method using 27F (5'-AGAGTTTGATCMTGGCTCAG-3') (SEQ ID NO: 1) and 1492R (5' - TACGGYTACCTTGTTACGACTT-3') (SEQ ID NO: 2) primers. The base sequencing of the corresponding site was requested to Macrogen Co., Ltd., and performed using 785F (5'-GGATTAGATACCCTGGTA-3') (SEQ ID NO: 3) and 907R (5'-CCGTCAATTCMTTTRAGTTT-3') (SEQ ID NO: 4) primers. The 16S rDNA sequencing identification of each strain completed by the base sequencing was performed using a 16S database in an NCBI BlastN (https://blast.ncbi.nlm.nih.gov/Blast) service. As a 16S rDNA sequencing standard identification result, ID-A05 was identified and analyzed with *Bacillus subtilis* at similarity of 99%. However, because it is difficult to accurately distinguish *B. subtilis* from similar species, such as *B. siamensis, B. velezensis,* and *B. amyloliquefaciens,* based on the 16S rDNA sequence, a more detailed identification analysis was performed through whole genome analysis.

### Example 3: Whole-genome sequencing of Bacillus subtilis and identification using whole genome

Whole-genome sequencing was requested to Macrogen Co., Ltd., and a genomic DNA was extracted using an DNeasy Blood & Tissue Kit (Qiagen, Germany), and then hybrid sequencing was performed using PacBio RS II (Pacific Biosciences, USA) and Illumina MiSeq (Illumina, USA).

The identification analysis using whole-genome information was performed by Average nucleotide identity (ANI) analysis using whole-genome sequences of *B. amyloliquefaciens* DSM 7(FN597644.1), *B. subtilis* NCIB 3610(CP034484.1), *B. siamensis* SCSIO 05746(CP025001.1), and *B. velezensis* FZB42(CP000560.2) of which whole-genome sequencing was completed conventionally to be registered in an NCBI Genome Database and whole-genome sequences of ID-A01, ID-A02, ID-A03, and ID-A04 of which whole-genome sequencing was completed. The ANI analysis was performed using a Blast algorithm (ANIb) in JspeciesWS (http://jspecies.ribohost.com/jspeciesws/). As a result of analyzing ANIb using the whole genome sequence, ID-A05 strain showed 76.28%, 98.79%, 76.37%, and 76.32% homology with the comparative strains, respectively. Through this analysis, strain ID-A05 was found to be *Bacillus subtilis.*

Novel *Bacillus subtilis* ID-A05 strain (Accession No.: KCTC14184BP) according to the present invention were deposited on May 11, 2020 to the Korean Biological Resources Center (KBRC).

### Example 4: Preparation of samples

**[**144] The respective strains were inoculated in a TSB medium and cultured for 40 hours at 37°C and 200 rpm. The culture medium was centrifuged (13,000 rpm, 20 min, 4°C) and then a supernatant was taken by removing the cells and then antibacterialized using a 0.22 µm filter to prepare samples.

### Example 5: Antibacterial efficacy evaluation (periodontal diseases)

The antibacterial activity was tested on *Porphyromonas gingivalis* (PGI), representative causative bacteria of periodontal diseases. The PGI strain used in this experiment was purchased *P. gingivalis* KCTC 5352 (type strain) from the Korean Collection for Type Cultures (KCTC) of the KRIBB. The PGI was anaerobic-cultured for 37°C for 24 hours in a TYB medium. The PGI and the samples were inoculated in the TYB medium by 10% and cultured for 24 hours and then absorbance at 600 nm was measured.

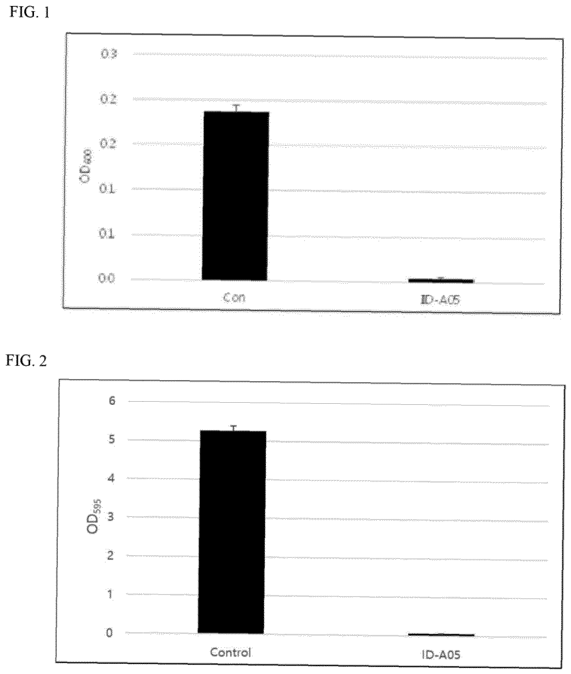

As a result, as shown in FIG. 1 below, the absorbance was measured to be very low in the medium treated with the strain ID-A05 culture supernatant, confirming that the antibacterial effect was excellent.

### Example 6: Biofilm efficacy evaluation - Prevention model (periodontal diseases)

The biofilm efficacy evaluation was performed on samples of strains which had the most excellent effect in the *Porphyromonas gingivalis* (PGI) antibacterial evaluation. The evaluation method was performed by a prevention model method in which a medium was dispensed in a 96 well plate coated with the saliva (4 hr, 37°C), inoculated with *Porphyromonas gingivalis* (PGI) and *Fusobacterium nucleatum* (FNU) at concentrations of 25% and 0.5%, respectively, treated with the samples at the same time, and then anaerobic-cultured for 37°C for 4 days, and then the results thereof were confirmed.

The formed biofilm was dyed with a 0.1% crystal violet reagent and the absorbance at 595 nm was measured. The more the biofilm was formed, the higher the absorbance was shown, so that the biofilm inhibitory effect of the test samples was confirmed through the absorbance comparison.

As a result, as shown in FIG. 2 below, the absorbance was measured low in the medium treated with the strain ID-A05 culture supernatant, confirming that the biofilm production inhibitory effect was excellent.

### Example 7: Measurement of biofilm inhibitory effect (periodontal diseases) using Scanning Electronic Microscope (SEM)

For biofilm induction, t a 24 well plate put with a coverslip was coated with saliva (4 hr, 37°C). A medium was dispensed in each well and inoculated with *Porphyromonas gingivalis* (PGI) and *Fusobacterium nucleatum* (FNU) at concentrations of 25% and 0.5%, respectively. The samples were treated at the same time and then anaerobic-cultured at 37°C for 4 days to induce the formation of the biofilm. The medium ingredients of each test well formed with the biofilm were removed and then freeze-dried to manufacture and capture an SEM sample.

### Example 8: Biofilm efficacy evaluation - Treatment model (periodontal diseases)

A medium was dispensed in a 96 well plate coated with the saliva (4 hr, 37°C) and then inoculated with *Porphyromonas gingivalis* and *Fusobacterium nucleatum* at concentrations of 25% and 0.5%, respectively, and anaerobic-cultured at 37°C for 4 days to form sufficiently a biofilm. Thereafter, the sample was treated while replacing the medium and further anaerobic-cultured at 37°C for 3 days, and the results thereof were confirmed. The formed biofilm was dyed with a 0.1% crystal violet reagent and the absorbance at 595 nm was measured. The more the biofilm was formed, the higher the absorbance was shown, so that the biofilm removal effect of the test samples was confirmed through the absorbance comparison.

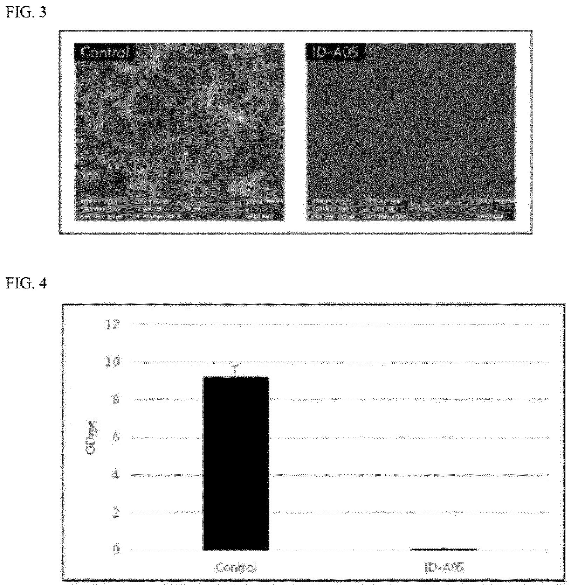

As a result, as shown in FIG. 4 below, the absorbance was measured low in the medium treated with the culture supernatant of the strain 1D-A05 and it was confirmed that the biofilm removal effect was excellent.

### Example 9: Anti- inflammatory efficacy evaluation (periodontal diseases)

In periodontitis or oral health, it was known that lipopolysaccharide (LPS) secreted from *Porphyromonas gingivalis* secreted inflammatory cytokines IL-1β, IL-6 and TNF-α. In order to measure the inflammatory cytokine secretion inhibitory ability of a candidate group, HGF-1 cells were seeded with 1×10⁴ cells/well in a 96 well plate and cultured for 24 hours. The candidate group was treated for 24 hours and then the LPS was treated with 50 µg /ml for 24 hours, and a supernatant was collected to be used for analysis. The amount of secreted cytokine was tested according to the manual using an ELISA assay kit and was measured at a plate reader 450 nm.

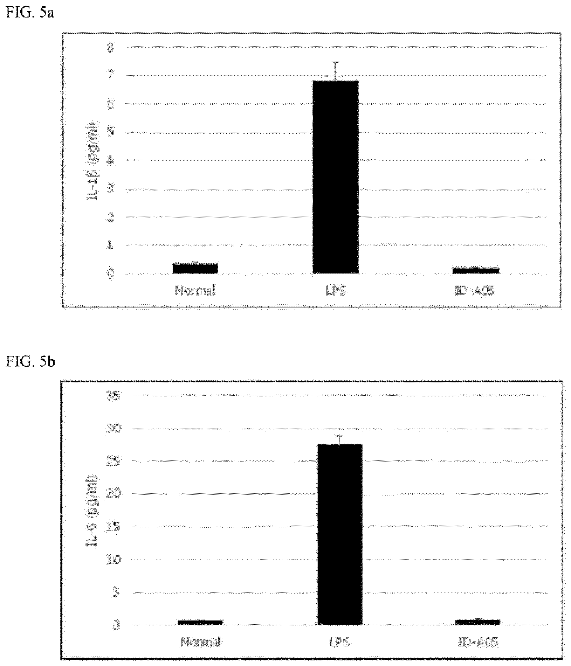

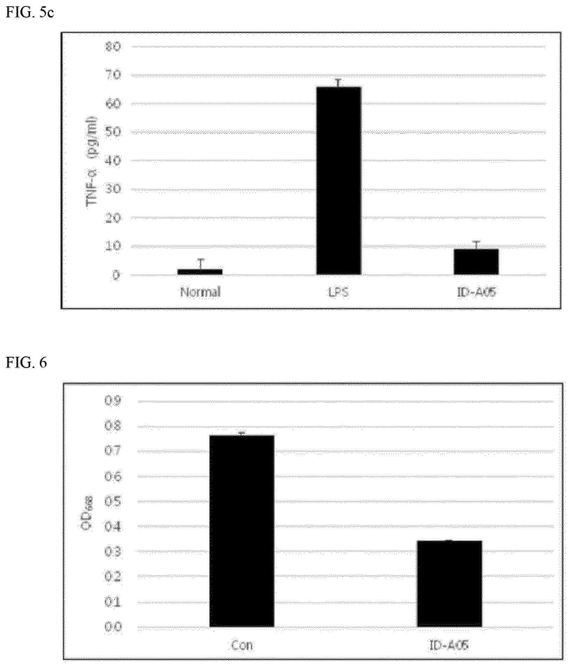

As the test result, as illustrated in FIGS. 5a, 5b, and 5c, it was confirmed that the secretion of cytokines by LPS was inhibited when the candidate group was treated. In addition, it was confirmed that all three cytokines, IL-1β, IL-6, and TNF-α, were reduced when the candidate group was treated.

### Example 10: Evaluation of Volatile sulfur compounds (Halitosis)

Volatile sulfur compounds (VSCs) included hydrogen sulfide (H₂S), methyl mercaptan (CH₃SH), dimethylsulfide (CH₃SCH₃), and the like as main causes of halitosis and among them, H₂S and CH₃SH accounted for 90% or more. The VSCs were mainly generated by causative bacteria of periodontal diseases and had a high concentration in patients with periodontal diseases. In order to confirm the effect on halitosis, the VSC generation inhibitory ability was tested. The pathogens were causative bacteria of periodontal diseases, and used with *Porphyromonas gingivalis* (PGI) and *Fusobacterium nucleatum* (FNU) strains with VSC generation ability. As the FNU strain, a type strain, *F. nucleatum* KCTC 2640 was purchased from the Korean Collection for Type Cultures (KCTC) of the KRIBB. The PGI and the FNU were inoculated in TYB and RCB media, respectively, and cultured in an anaerobic chamber at 37°C for 24 hours. 5% of the PGI and the FNU and 10% of the sample were inoculated in a medium mixed with the TYB and RCB media at 1:1 and cultured at 37°C for 24 hours. In order to measure the generated H₂S after culturing, the medium was color-developed through a methylene blue method and then the absorbance at 668 nm was measured.

As a result, as shown in FIG. 6 below, the absorbance was measured low in the medium treated with the culture supernatant of the strain ID-A05, and it was confirmed that the volatile sulfur compound production inhibitory effect was excellent.

### Example 11: Antibacterial efficacy effect (cavities)

The antibacterial activity on *Streptococcus mutans* (SMU) as a main causative bacteria of cavities was tested. As the SMU strain used in the test, *Streptococcus mutans* KCTC 3065 as a type strain was received from the Korean Collection for Type Cultures (KCTC) of the KRIBB. The SMU was cultured at a BHI medium at 37°C in the CO₂ incubator for 24 hours. The SMU and the samples were inoculated by 10% in the BHI medium and cultured for 24 hours, and then the viable cell count analysis was performed.

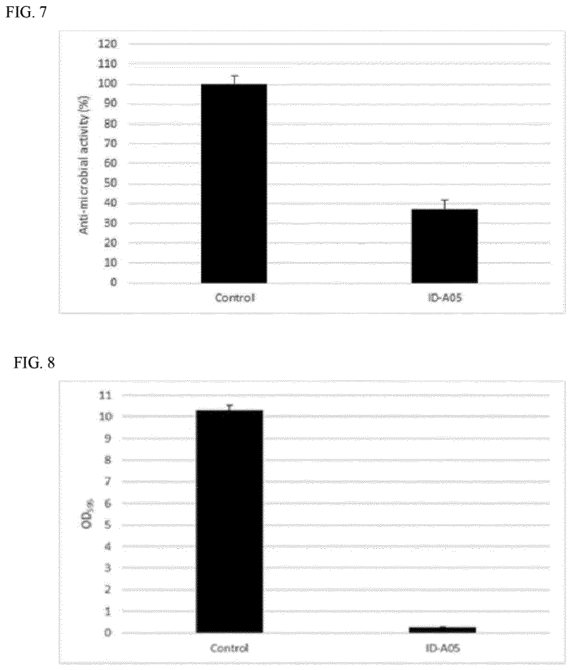

As a result, as shown in FIG. 7 below, the absorbance was measured to be very low in the medium treated with the culture supernatant of the strain ID-A05, confirming that the antibacterial effect was excellent.

### Example 12: Biofilm efficacy evaluation (cavities)

For biofilm induction, *Streptococcus mutans* (SMU) inoculated by 10% in the BHI was cultured (5% CO₂ incubator, 18 hours). The culture medium was centrifuged (9,000 rpm, 5 mm, 4°C) to secure the bacteria, diluted at about 1 x 10⁶ CFU/mL in reaction media (BHI with 1% Sucrose), and the bacteria solution were seeded with to include 10% (v/v) of samples. The media change to reaction media including 10% (v/v) samples was performed every 24 hours and the film formation was induced up to 3 days (72 hours) as an appropriate biofilm formation date under the corresponding condition.

For biofilm efficacy evaluation, a crystal violet staining method was performed. After fixation with methyl alcohol and staining with 0.1% crystal violet, the reconstruction was performed with ethyl alcohol. A washing process was included for each step and a film formation degree at 595 nm was measured.

As a result, as shown in FIG. 8 below, the absorbance was measured very low in the medium treated with the culture supernatant of the strain ID-A05, confirming that the biofilm inhibitory effect was excellent.

### Example 13: Measurement of biofilm inhibitory effect (cavities) using Scanning Electronic Microscope (SEM)

For **biofilm** induction, SMU inoculated by 10% in the BHI was cultured (5% CO₂ incubator, 18 hours). The culture medium was centrifuged (9,000 rpm, 5 min, 4°C) to secure the bacteria, diluted at about 1 x 10⁶ CFU/mL in reaction media (BHI with 1% Sucrose) and prepared. In order to facilitate metal coating as a pretreatment process required for SEM measurement, a cover slip was laid on a plate well performing a test to induce a biofilm on the cover slip, and the diluted SMU was seeded in each well to include 10% (v/v) of the samples. The media change to reaction media including 10% (v/v) samples was performed every 24 hours and the film formation was induced up to 3 days (72 hours) as an appropriate biofilm formation date under the corresponding condition. Thereafter, the cover slip induced with the biofilm was washed to be transferred to a new plate and then a freeze-drying process was performed for SEM measurement.

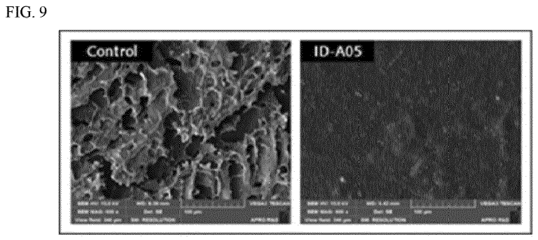

As a result, as shown in FIG. 9 below, it was confirmed that the degree of biofilm formation was very low in the cover slip treated with the strain ID-A05 - derived culture supernatant, and the antibacterial effect was excellent.

### INDUSTRIAL APPLICABILITY

According to the present invention, the composition comprising the *Bacillus subtilis* ID-A05 strain, the culture media or culture supernatants thereof provided in the present invention exhibit antibacterial, anti-inflammatory, and biofilm inhibitory effects on causative bacteria of cavities and periodontal diseases to be used for treating or preventing cavities and periodontal diseases or improving halitosis, and thus industrial applicability is very excellent.

### Accession number

Accession Institute Name: Korea Research Institute of Bioscience and Biotechnology (KRIBB)
Accession number: KCTCI4184BP
Accession Date: 20200511
Accession Institute Name: KRIBB, 181, Ipsin-gil, Jeongeup-si, Jeollabuk-do, Korea (56212)

### BUDAPEST TREATY ON THE INTERNATIONAL RECOGNITION OF THE DEPOSIT OF MICROORGANISMS FOR THE PURPOSES OF PATENT PROCEDURE INTERNATIONAL FORM RECEIPT IN THE CASE OF AN ORIGINAL DEPOSIT issued pursuant to Rule 7.1

To. Yoon, Woong Seop

Ildong Pharmaceutical, 2, Baumoe-ro 27-gil, Seocho-gu, Seoul, Korea

| I. IDENTIFICATION OF THE MICROORGANISM | |
|---|---|
| Identification reference given by the DEPOSITOR: | Accession number given by the INTERNATIONAL DEPOSITARY AUTHORITY: |
| *Bacillus subtilis* ID-A05 | KCTC 14184BP |
| II. SCIENTIFIC DESCRIPTION DESIGNATION AND/OR PROPOSED TAXONOMIC | |
| The microorganism identified under I above was accompanied by: | |
| □ a scientific description | |
| □ a proposed taxonomic designation | |
| (Mark with a cross where applicable) | |
| III. RECEIPT AND ACCEPTANCE | |
| This International Depositary Authority above, which was received by it on May accepts the microorganism identified under I 11, 2020. | |
| IV. RECEIPT OF REQUEST FOR CONVERSION | |
| The microorganism identified under I above was received by this International Depositary Authority on and a request to convert the original deposit to a deposit under the Budapest Treaty was received by it on. | |
| V. INTERNATIONAL DEPOSITARY AUTHORITY | |
| Name: Korea Collection for Type Cultures | Signature(s) of person(s) having the power to represent the International Depositary Authority or of authorized official(s): |
| Address: Korea Research Institute of Bioscience and Biotechnology (KRIBB), 181, Ipsin-gil, Jeongeup-si, Jeollabuk-do, Korea (56212) | Kim, Sung Gun center director |
| | Date: May 11, 2020 |

Form BP/4

Sole page

(It is confirmed that there is no difference from the original text.)

Agent: Patent Attorney Lee, Hee Sook, Patent Attorney Kim, Seok Man

## Claims

1. A *Bacillus subtilis*ID-A05 strain (Accession No.: KCTC14184BP).

2. The *Bacillus subtilis* strain of claim 1, wherein the strain is excellent in i) an effect of treating or preventing cavities and periodontal diseases; and ii) an effect of improving halitosis.

3. A culture medium of the *Bacillus subtilis* strain of claim 1 or 2

4. A culture supernatant of the *Bacillus subtilis* strain of claim 1 or 2

5. A pharmaceutical composition for preventing or treating cavities, periodontal diseases or halitosis comprising a *Bacillus subtilis* strain of claim 1, a culture medium thereof, or a culture supernatant thereof as an active ingredient.

6. The pharmaceutical composition of claim 5, wherein the periodontal diseases are at least one selected from the group consisting of gingivitis, periodontal inflammation, gingival bleeding, gingival retraction, periodontal pocket, pericoronitis and periodontal abscess.

7. A quasi-drug composition for preventing or improving cavities, periodontal diseases or halitosis comprising a *Bacillus subtilis* strain of claim 1, a culture medium thereof, or a culture supernatant thereof as an active ingredient.

8. A health functional food composition for preventing or improving cavities, periodontal diseases or halitosis comprising a *Bacillus subtilis* strain of claim 1, a culture medium thereof, or a culture supernatant thereof as an active ingredient.

9. A food composition for preventing or improving cavities, periodontal diseases or halitosis comprising a *Bacillus subtilis* strain of claim 1, a culture medium thereof, or a culture supernatant thereof as an active ingredient.

10. A toothpaste composition for preventing or improving cavities, periodontal diseases or halitosis comprising a *Bacillus subtilis* strain of claim 1, a culture medium thereof, or a culture supernatant thereof as an active ingredient.

11. A mouth washer composition for preventing or improving cavities, periodontal diseases or halitosis comprising a *Bacillus subtilis* strain of claim 1, a culture medium thereof, or a culture supernatant thereof as an active ingredient.

12. A gargle composition for preventing or improving cavities, periodontal diseases or halitosis comprising a *Bacillus subtilis* strain of claim 1, a culture medium thereof, or a culture supernatant thereof as an active ingredient.

13. Use of a *Bacillus subtilis* strain of claim 1, a culture medium thereof, or a culture supernatant thereof for preparing an agent for treating cavities, periodontal diseases or halitosis.

14. Use of claim 13, wherein the periodontal diseases are at least one selected from the group consisting of gingivitis, periodontal inflammation, gingival bleeding, gingival retraction, periodontal pocket, pericoronitis and periodontal abscess.

15. A method of treating cavities, periodontal diseases or halitosis comprising administering an effective amount of a composition containing a *Bacillus subtilis* strain of claim 1, a culture medium thereof, or a culture supernatant thereof to a subject in need thereof.

16. A method of claim 15, wherein the periodontal diseases are at least one selected from the group consisting of gingivitis, periodontal inflammation, gingival bleeding, gingival retraction, periodontal pocket, pericoronitis and periodontal abscess.
